# EUROPÄISCHE PATENTSCHRIFT

(11) **EP 1 521 759 B1**
(45) Veröffentlichungstag und Bekanntmachung des Hinweises auf die Patenterteilung: **30.11.2011**
(21) Anmeldenummer: 03762464.0
(22) Anmeldetag: 04.07.2003
(51) Int. Cl.: C07H 1/00

(54) **MIT NUKLEINSÄUREN BESCHICHTETER METALLISCHER GEGENSTAND, VERFAHREN ZU DESSEN HERSTELLUNG UND DESSEN VERWENDUNG**
METALLIC OBJECT WITH A NUCLEIC ACID COATING AND METHOD FOR PRODUCING SAID OBJECT
OBJET METALLIQUE RECOUVERT D'ACIDES NUCLEIQUES, SON PROCEDE DE PRODUCTION ET D'UTILISATION

(30) Priorität: 04.07.2002 DE 10232139
(43) Veröffentlichungstag der Anmeldung: 13.04.2005
(73) Patentinhaber: Technische Universität Dresden, 01069 Dresden (DE)
(72) Erfinder: SCHARNWEBER, Dieter, 01324 Dresden (DE); BEUTNER, Rene, 01217 Dresden (DE); ROESSLER, Sophie, 01156 Dresden (DE); HANKE, Thomas, 13187 Berlin (DE); WORCH, Hartmut, 01187 Dresden (DE); SCHWENZER, Bernd, 01728 Bannewitz (DE); MICHAEL, Jan, 01237 Dresden (DE)
(74) Vertreter: Uhlemann, Henry
(86) Internationale Anmeldenummer: PCT/DE2003/002305
(87) Internationale Veröffentlichungsnummer: WO 2004/005306

(56) Entgegenhaltungen:
- EP-A- 0 391 608
- WO-A1-03/086495
- WO-A1-03/086495
- DE-A- 4 309 248
- DE-A- 19 643 555
- [Online] Gefunden im Internet: <URL:http://en.wikipedia.org/wiki/DNA>
- [Online] Gefunden im Internet: <URL:http://en.wikipedia.org/wiki/RNA>
- [Online] Gefunden im Internet: <URL:http://en.wikipedia.org/wiki/Nukleinsa uren>
- [Online] Gefunden im Internet: <URL:http://en.wikipedia.org/wiki/DNA> [gefunden am 2009-04-21]
- [Online] Gefunden im Internet: <URL:http://en.wikipedia.org/wiki/RNA> [gefunden am 2009-04-21]
- [Online] Gefunden im Internet: <URL:http://de.wikipedia.org/wiki/Nukleinsä uren> [gefunden am 2009-04-21]

## Beschreibung

Die Erfindung betrifft einen metallischen Gegenstand mit einer stabilen Beschichtung aus Nukleinsäuren und/oder Nukleinsäurederivaten und ein Verfahren zur Herstellung genannter Beschichtung. Durch die Kopplung von Wirkstoffen an die Nukleinsäuren und/oder Nukleinsäurederivate kann die Beschichtung an verschiedenste Anwendungen angepasst und die Biokompatibilität entsprechend modifizierter Oberflächen erhöht werden.
Derartig beschichtete Metalle sind interessant für die Medizin und Tiermedizin, z. B. für Implantate, aber auch für verschiedenste Bereiche der Biotechnologie sowie der Chip- und Sensortechnologie.

Eine effiziente Immobilisierung von Nukleinsäuren und Nukleinsäurederivaten auf festen Trägermaterialien ist von großer Bedeutung für viele Bereiche der Biotechnologie.
So sind Verfahren zur Immobilisierung von Nukleinsäuren auf Glas, z.B. zur Herstellung von Nukleinsäurearrays, sowie zur Immobilisierung auf Goldpartikeln (US 6291188 B1, EP 1170374 A1) z. B. für den Gentransfer bekannt. Die Immobilisierung auf Goldpartikeln geschieht dabei entweder durch Adsorption (EP 1170374 A1) oder über einen Schwefellinker (US 6291188 B1).

Des Weiteren sind Verfahren zur Adsorption von Nukleinsäuren an Zirkonium(IV)-oxid und Aluminiumoxid und anderen Metalloxiden beschrieben (DE 4309248 A1, EP 0391 608 A2, WO 92/18514 A1). Um diese Adsorption an oben genannte Metalloxide zu gewährleisten, muss das Material allerdings zu mindestens 50%, vorzugsweise 99% aus Metalloxid bestehen (EP 0391 608 A2, Seite 4, Zeilen 18 - 29).

Für zahlreiche Anwendungen ist eine orientierte, reproduzierbare, dauerhafte, feste, chemikalien- und temperaturresistente Verbindung der Nukleinsäuren und/oder Nukleinsäurederivate mit der Substratoberfläche wünschenswert.

Beschrieben sind auch Verfahren zur Bindung von Oligonukleotiden an Ventilmetallen, wie silanisiertes Tantal (Bier, FF und Scheller, FW. 1996. Biosensors & Bioelectronics 11:669-674) und silanisiertes Titan (Bier, FF et al. 1999. Biotechniques 27:752-760). Um die für diese Verfahren notwendige Silanisierung der Metalloberfläche zu erreichen, muss das Metall unter aggressiven chemischen Bedingungen vorbehandelt werden (s. auch Xiao, SJ et al. 1998. Langmuir 14:5507-5516). Nachteilig bestehen diese Verfahren aus mindestens drei Schritten. Zusätzlich müssen auch die Nukleinsäuren dazu aufwendig modifiziert werden.

Bekannt sind Methoden, die eine feste Verbindung von organischen Molekülen, wie z. B. Collagen, auf Ventilmetall-Oberflächen gewährleisten (DE 19643555). Auf solchen Metallen bzw. Legierungen ausgebildete Oxidschichten weisen zumindest bei anodischer Polarisation Ionenleitung auf und erlauben damit über anodische Polarisation eine Variation der Oxidschichtdicke in weiten Grenzen. Dazu wird das Phänomen ausgenützt, dass bei anodischer Polarisation in wässrigen Lösungen die bereits vorhandene Oxidschicht auf Titanwerkstoffen durch Wanderung von Ionen im elektrischen Feld zu wachsen beginnt. In diese wachsende Schicht können an der Oberfläche z. B. durch Adsorption vorhandene Moleküle oder funktionelle Gruppen in die Oxidschicht eingebaut werden. Ein orientierter Einbau über definierte Molekülbereiche (regiospezifischer Einbau) von Substanzen und damit der Erhalt bestimmter Eigenschaften/Funktionen der Substanzen ist dabei jedoch nicht zu realisieren.

Von anorganischen Anionen, vor allem von Phosphat, ist bekannt, dass sie in solche anodisch wachsende Titan(IV)oxid-Schichten eingebaut werden können. Dabei kann das Anion unter Umständen auch als funktionelle Gruppe ein Teil von größeren Molekülen sein (DE19643555). Damit das die anionischen Gruppen tragende Molekül in die anodischen Oxidschichten eingebaut wird, müssen die elektrostatischen Ladungsverhältnisse zwischen Oberfläche und adsorbierendem Molekül(teil) eine primäre Adsorption zulassen.

Negativ-geladene Moleküle, wie Nukleinsäuren, werden allerdings von der, unter physiologischen Bedingungen negativ-geladenen, Titan-Titan(IV)oxid-Oberfläche abgestoßen. Die in DE19643555 beschriebene Methode ist aus diesem Grunde nicht auf Nukleinsäuremoleküle anwendbar.

WO 03/086495 A (nachveröffentlicht) offenbart eine medizinische Prothesevorrichtung umfassend ein Metallmaterial (A) Titan, Zirkon, Tantal, Niob oder deren Legierungen oder eine Chrom-Vanadium-Legierung, wobei Oberflächenabschnitte des metallischen Materials (A) mit einer Hydroxidschicht (B) beschichtet sind.

Ein genereller Nachteil der bisher in Medizin, Biologie und Chemie verwendeten, chemisch oder biochemisch modifizierten Oberflächen ist der Mangel an Variabilität und Modularität. Einmal aufgebrachte Substanzen verbleiben vielfach irreversibel an den Oberflächen oder weisen eine nur bedingt einstellbare Freisetzungskinetik auf. Zudem muss das Beschichtungsverfahren meist aufwendig an die gewünschte Beschichtung angepasst werden. Eine Anpassung der Beschichtung durch den Anwender an seine Einsatzzwecke ist dadurch nahezu unmöglich.

Die Aufgabe der Erfindung ist es, einen metallischen Gegenstand mit einer stabilen Beschichtung aus Nukleinsäuren und/oder Nukleinsäurederivaten zu schaffen, bei dem die Nukleinsäuren für weitere Reaktionen, wie z. B. Hybridisierungen optimal zugänglich sind.

Erfindungsgemäß wird die Aufgabe gelöst durch einen metallischen Gegenstand nach Anspruch 1, dessen Oberfläche mit einer dünnen Metalloxidschicht überzogen und mit Nukleinsäuren beschichtet ist, wobei 5'- oder 3'- terminale Molekülbereiche von Nukleinsäuren durch Integration in eine wachsende Metalloxidschicht stabil in die Metalloxidschicht eingebaut sind, wobei die eingebauten 5' oder 3' terminalen Bereiche der Nukleinsäuren anionischen Gruppen tragen und durch den regiospezifischen Einbau der terminalen Molekülbereiche eine frei bewegliche Orientierung der nicht-eingebauten Bereiche der Nukleinsäuren zur Metalloberfläche erreicht wird.

Unter dünner Metalloxidschicht wird dabei verstanden, dass die Metalloxidschicht zumindest dünner ist als der mittlere Durchmesser des metallischen Gegenstands.

Wenn nachfolgend von Nukleinsäuren die Rede ist, schließt dieser Begriff grundsätzlich Nukleinsäurederivate mit ein.
Insbesondere sind im Sinne dieser Beschreibung unter Nukleinsäuren folgende Moleküle zu verstehen: Desoxyribonukleinsäuren (DNA), Ribonukleinsäuren (RNA) und Peptid-Nukleinsäuren (PNA), sowie auch alle aus diesen Grundstrukturen ableitbaren Modifikationen wie z. B. Phosphothioate, Phosphoramidate, O-Methyl-Derivate und locked nucleic acids (LNA). Die Nukleinsäuren können dabei Einzelstränge, Doppelstränge oder daraus gemischte Strukturen sein. Vorzugsweise ist auch bei einer Beschichtung mit doppelsträngiger DNA nur ein Nukleinsäurestrang fest mit der Metall-Metalloxid-Oberfläche (Substratoberfläche) verbunden.

Der stabile Einbau der 5'- oder 3'-terminalen Molekülbereiche in die Metalloxidschicht wird durch das erfindungsgemäße Verfahren erreicht, bei dem elektrochemisch um die Molekülbereiche herum eine Metalloxidschicht aufgebaut wird. Der stabile Einbau ergibt sich durch die Integration der terminalen Molekülbereiche in die wachsende Metalloxidschicht.

Durch diesen regiospezifischen Einbau der terminalen Molekülbereiche, wird eine frei bewegliche Orientierung der nicht-eingebauten Bereiche der Nukleinsäuren zur Metalloberfläche erreicht. Durch die freie Beweglichkeit sind die nicht eingebauten Bereiche der Nukleinsäuren vorteilhaft für anschließende Prozesse, wie eine Hybridisierung mit einem komplementären Strang, optimal zugänglich.

Ein weiterer Vorteil ist, dass alle immobilisierten Nukleinsäuremoleküle derart regiospezifisch in die Oxidschicht eingebaut sind. Dadurch ergibt sich eine gleichmäßig orientiert beschichtete Oberfläche.
Dieser regiospezifische, reproduzierbare und feste Einbau der terminalen Molekülbereiche in die Metalloxidschicht ist durch die nach dem Stand der Technik bekannte Adsorption von Nukleinsäuren nicht erreichbar.

Erfindungsgemäß tragen die eingebauten 5'- oder 3'-terminalen Bereiche der Nukleinsäuren anionische Gruppen, vorzugsweise Phosphat, Phosphonat oder Sulfonat.

Der metallische Gegenstand besteht vorzugsweise aus einem Ventilmetall, wie z. B. Aluminium, Titan, Tantal, Zirkonium, Niob oder deren Legierungen, einschließlich intermetallischer Phasen.

Die Sequenzen der an den 5'-terminalen Molekülbereichen immobilisierten Nukleinsäuren werden vorteilhaft so ausgewählt, dass die für anschließende Prozesse, wie z. B. Hybridisierung, relevanten Sequenzen auch bei einem Schichtwachstum von über 2 nm noch zugänglich sind. Die Sequenzen in der direkten Nähe des 5'-Terminus sind dabei beliebig, da diese in die Oxidschicht eingebaut werden. Wohingegen die Sequenzen in der Nähe der freibeweglichen 3'-terminalen Molekülbereichen, vorteilhaft spezifische Erkennungssequenzen, z. B. für eine anschließende Hybridisierung mit komplementären Strängen, enthalten.
Gleiches gilt entsprechend für über die 3'-terminalen Molekülbereiche immobilisierten Nukleinsäuren.

In einer besonders vorteilhaften Ausführungsform des erfindungsgemäßen metallischen Gegenstand, an dessen Oberfläche einzelsträngige Nukleinsäuren immobilisiert sind, sind über Watson-Crick- und andere Basenpaarungen komplementäre Nukleinsäure-Einzelstrangmoleküle an diese gebunden. Die Sequenz des immobilisierten Stranges bestimmt dabei, welche Sequenz(en) an diesen komplementär binden können.

Erfindungsgemäß können an die komplementär-bindenden Nukleinsäurestränge verschiedenste biologische oder chemische Wirkstoffe kovalent gebunden sein. Vorteilhaft werden dabei die biologischen bzw. chemischen Eigenschaften der entsprechend modifizierten Oberfläche weitgehend von den an die Nukleinsäuren gekoppelten Wirkstoffen bestimmt.
Die Bindung der komplementären Stränge kann vorteilhaft auch vom Anwender durchgeführt werden. Durch die Auswahl komplementärer Stränge mit geeigneten Wirkstoffen kann der Anwender die biologischen bzw. chemischen Eigenschaften der Metalloberfläche an die Bedürfnisse, die seine spezielle Anwendung erfordert, anpassen.
Die Modularität dieses Systems garantiert demnach eine nahezu uneingeschränkte Variabilität beim Aufbau "biologisierter" Oberflächen.

Die Wirkstoffe sind beispielsweise anorganische oder organische oder biochemische Moleküle oder Zell- oder Gewebekomponenten.

Dabei können, ausgehend von einem metallischen Gegenstand, der mit einzelsträngigen Nukleinsäuren beschichtet ist, die biologischen Eigenschaften der Oberfläche durch folgende Faktoren vielseitig variiert werden:
- Die Sequenzen der immobilisierten Stränge bestimmen, welche Nukleinsäurensequenzen komplementär binden können.
- Die Stabilität der Bindung zwischen immobilisierten und komplementären Strängen bestimmt das Freisetzungsverhalten und die resultierende Bioverfügbarkeit an letztere gebundener Wirkstoffe.
- An immobilisierte Stränge mit gleicher Sequenz können verschiedene Wirkstoffe tragende komplementäre Stränge binden.
- Auf den immobilisierten Strängen können lateral definiert oder statistisch verteilt verschiedene Nukleinsäuren gleichzeitig immobilisiert werden, bei gleicher Flexibilität in der Gestaltung der in den drei Punkten zuvor beschriebenen Eigenschaften.
- Durch die variable Gestaltung der Hybridstabilität auf ein und derselben Oberfläche kann das Freisetzungsverhalten der assoziativ gebundenen und Wirkstoff-tragenden komplementären Nukleinsäuren gesteuert werden.
- Durch Modifizierung der Basen, wie beispielsweise Inosin, bzw. des Rückgrats der Nukleinsäuren, wie beispielsweise ein Peptid-, Phospothioat- oder Methylphosphonat-Rückgrat, kann die chemische Stabilität und die Stabilität gegenüber dem Abbau durch Nukleasen gezielt beeinflusst werden. Die oben genannten Eigenschaften werden dadurch nicht maßgeblich beeinträchtigt.
- Die Sequenz der immobilisierten Nukleinsäuren kann spezielle Erkennungssequenzen für Antikörper, Nukleinsäure-bindende Proteine oder Nukleasen enthalten.

Die Stabilität der Bindung zwischen immobilisierten und komplementären Strängen ist dabei im Wesentlichen abhängig von der Länge des Hybridbereiches, dessen G-C-Gehalt und der Anzahl von sogenannten "Mismatches", d. h. nicht komplementären Basenpaaren. Dabei steigt die Stabilität mit dem G-C-Anteil, durch eine größere Zahl von Wasserstoffbrücken (G-C-Paare: 3 Wasserstoffbrücken; A-T-Paare: 2 Wasserstoffbrücken). Mismatch-Paare bilden keine Wasserstoffbrücken aus, da sie nicht komplementär sind. Je größer die Zahl der Mismatches, desto instabiler ist das Hybrid.
Ist die Freisetzung der komplementären Stränge und der an diese gekoppelten Wirkstoffe nicht erwünscht, kann diese vorteilhaft durch kovalente Bindung des komplementären Strangs an den immobilisierten Strang verhindert werden. Eine solche kovalente Bindung wird z. B. durch Vernetzung mit UV-Licht oder chemische Reaktionen erreicht.
Ist eine besonders schnelle Freisetzung der, an die komplementären Stränge gebundenen, Wirkstoffe in einem biologischen Milieu erwünscht, kann dies durch den Einbau von Erkennungssequenzen für Nukleasen erreicht werden.

Der erfindungsgemäß mit Nukleinsäuren beschichtete Gegenstand grenzt sich durch folgende wesentliche Vorteile von Gegenständen, die mittels anderer Methoden mit Nukleinsäuren beschichtet sind, ab:
- Die Stabilität der Nukleinsäure-Beschichtung.
- Durch die regiospezifische Fixierung der Nukleinsäuren an den terminalen Molekülbereichen ergibt sich eine optimale Zugänglichkeit für anschließende Prozesse, wie beispielsweise die Hybridisierung.
- Die Stabilität der Immobilisierung und die Länge der nicht-eingebauten Bereiche der Nukleinsäuren sind abhängig von der Dicke der Metalloxidschicht, in die die terminalen Molekülbereiche der Nukleinsäuren eingebaut sind. Diese Schichtdicke kann vorteilhaft mit dem erfindungsgemä-βen Verfahren gesteuert werden.
- Möglichkeit zur variablen und modularen Modifizierung der Metalloberfläche durch Binden von komplementären Strängen und an diese gebundene Wirkstoffe,
- Möglichkeit der Beeinflussung des Freisetzungsverhaltens und damit der Bioverfügbarkeit der Wirkstoffe durch die molekulare Struktur des Nukleinsäure-Hybrids,
- Möglichkeit der einfachen Anpassung der Beschichtung durch den Anwender.

Erfindungsgemäß wird der mit Nukleinsäuren beschichtete metallische Gegenstand durch ein Verfahren nach Anspruch 12 hergestellt, bei dem elektrochemisch um 5'- oder 3'-terminalen Molekülbereiche der Nukleinsäuren herum eine Metalloxidschicht aufgebaut wird. Dabei werden die an mindestens einem terminalen Molekülbereich (5'- oder 3'-terminalen Molekülbereich) anionische Gruppen tragenden Nukleinsäuren mit dem metallischen Gegenstand so in Kontakt gebracht werden, dass diese durch eine regiospezifische Wechselwirkung, nämlich durch eine adsorptive Anlagerung, zunächst metastabil auf der Metalloxidoberfläche vorliegen. Parallel dazu oder anschließend wird der metallische Gegenstand anodisch in einer Elektrolytlösung polarisiert. Durch anodische Polarisation bei pH 3 bis 6 wird eine mehrschichtige Oxidschicht erzeugt, in deren äußere Schicht die anionische Gruppen tragenden terminalen Molekülbereiche der Nukleinsäuren stabil eingebaut werden.

Überraschend wurde festgestellt, dass eine terminale Modifikation der Nukleinsäuren mit anionischen Gruppen geeigneten pKₛ-Wertes die Immobilisierung von Nukleinsäuren ermöglicht. Dabei bestehen die anionischen Gruppen aus Molekülstrukturen, die bei dem pH-Wert und der Ionenstärke des Verfahrens eine negative Ladung besitzen. Vorzugsweise sind die anionischen Gruppen Phosphat- oder Phosphonat- oder Sulfonatgruppen. Die Verbindung der anionischen Gruppen mit den terminalen Molekülbereichen erfolgt vorzugsweise kovalent an den 3'- oder 5'-Termini der Nukleinsäuren.
Vorteilhaft sind terminal (endständig) phosphorylierte Oligonukleotide kommerziell erhältlich.

Erfindungsgemäß beginnt der Einbau der Nukleinsäuren an der terminalen anionischen Gruppe und wird im Wesentlichen durch diese bestimmt. Im Unterschied zu terminalen Phosphatgruppen sind die Phosphatgruppen des Nukleinsäurerückgrats nicht geeignet, einen Einbau in die Metalloxidschicht zu initiieren (siehe Ausführungsbeispiel 4). Die Phosphatgruppen im DNA-Rückgrat können somit durch andere Bindungstypen, wie z. B. Peptid, Phosphothioat, MethylPhosphonat oder andere ersetzt werden.

Erfindungsgemäß werden die Bedingungen für die Immobilisierung so gewählt, dass die anionischen Gruppen noch mindestens eine negative Ladung tragen, während die Metall-Metalloxid-Oberfläche zumindest einige lokal positive Ladungszentren aufweist. Bei diesen Bedingungen wird eine elektrostatischinitiierte adsorptive Anlagerung der terminalen Bereiche der Nukleinsäuren an den positiven Ladungszentren der Oxidoberfläche ermöglicht Erfindungsgemäß erfolgt die adsorptive Anlagerung und der anschließende Einbau durch anodische Polarisation bei pH 3 bis 6, vorzugsweise bei pH 4 in Acetatpuffer.

Das Wachstum der Oxidschicht kann durch die Wahl der elektrochemischen Parameter, vorzugsweise Potenzial, Stromdichte und Potenzialänderungsgeschwindigkeit kontrolliert werden. Dabei bestimmen die elektrochemischen Parameter die auftretende und die Größe der Moleküle die zulässige Tiefe des Einbaus.

Vorzugsweise wird dabei für das erreichte Potenzial ein Wert zwischen 2 und 200 V_{SCE} gewählt. Dieses Potential gewährleistet vorteilhaft einen ausreichend stabilen Einbau der Nukleinsäuren in die Oxidschicht und verhindert gleichzeitig das Wachstum der Oxidschicht in einen, z. B. für eine spätere Hybridisierung, notwendigen Erkennungsbereich der Nukleinsäure.
Nach der Immobilisierung wird die erzeugte Matrix mit Puffer (ph ≈ 4) und destilliertem Wasser gespült. Die Probe kann sofort weiterverarbeitet oder getrocknet bei kühlen Temperaturen (<10°C) unter Lichtausschluss gelagert werden.

Überraschend ergibt sich als Resultat der erfindungsgemäßen Immobilisierung eine freie Zugänglichkeit der nicht-eingebauten Abschnitte der immobilisierten Nukleinsäurestränge. Dadurch ist der nicht-eingebaute Teil der Nukleinsäure für anschließende Prozesse, wie eine Hybridisierung mit einem komplementären Strang, optimal verfügbar.

Für eine mögliche anschließende Hybridisierung von komplementären Nukleinsäuresträngen an das mit einzelsträngigen Nukleinsäuren beschichtete Metall müssen die Bedingungen so gewählt werden, dass eine bestmögliche Wechselwirkung und damit die Bildung ausreichend stabiler Hybride zwischen den immobilisierten und den in Lösung befindlichen Einzelsträngen stattfindet.

Für die nach der Immobilisierung durchzuführende Hybridisierung werden der pH-Wert und die Ionenstärke so gewählt, dass sowohl die Metall-Metalloxid-Oberfläche als auch das Nukleinsäure-Rückgrat negativ geladen sind und somit eine elektrostatische Abstoßung zwischen dem DNA-Rückgrat und der Metall-Metalloxid-Oberfläche erreicht wird, die stabile Immobilisierung der Nukleinsäuren über ihren terminalen Einbau in die Oxidschicht jedoch erhalten bleibt. Im Falle von Nukleinsäurederivaten mit ungeladenem Rückgrat werden die Bedingungen so gewählt, dass die Metall-Metalloxid-Oberfläche negativ geladen ist. Durch die Abstoßung von dieser Oberfläche wird eine freibewegliche Orientierung der immobilisierten Stränge unterstützt und eine unspezifische Adsorption der komplementären Stränge, die zu einer Behinderung der Hybridisierung führen würde, vermieden.

In Abhängigkeit von Länge und Basenzusammensetzung der zu hybridisierenden Sequenzen wird ein Puffersystem geeigneter Ionenstärke, üblicherweise im Bereich zwischen 0,1 und 1,5 mol/Liter an einwertigen Kationen gewählt. Der pH-Wert der Hybridisierungslösung liegt erfindungsgemäß zwischen pH 4 bis pH 10, vorzugsweise zwischen pH 5,5 und pH 8,5, besonders vorzugsweise zwischen pH 7,0 und pH 7,5. In der Hybridisierungslösung befinden sich die Komplementärstrange, an die anorganische, organische, vorrangig bioaktive, Gruppen oder Moleküle gebunden sind. Diese Bindung ist vorzugsweise kovalenter Natur. In einer alternativen Ausführungsform erfolgt die Bindung von Molekülen an die Komplementärstränge über eine Biotin/Avidin- bzw. Streptavidinbrücke.

Für die Hybridisierung wird die Oberfläche des mit einzelsträngigen Nukleinsäuren beschichteten metallischen Gegenstandes zwischen 10 Minuten und 2 Stunden mit der beschriebenen Hybridisierungslösung inkubiert und anschlie-βend mit Pufferlösung und Wasser gespült.
Vorteilhaft sind die Bedingungen der Hybridisierung milde und können so gewählt werden, dass sie nahezu physiologischen Bedingungen entsprechen. Dadurch wird eine bestmögliche molekulare und funktionelle Unversehrtheit von biologischen Wirkstoffen, die an die komplementären Stränge gebunden sind, gewährleistet.
Der so beschichtete Gegenstand kann, falls keine unmittelbar anschließende Verwendung erfolgt, bei kühlen Temperaturen (<10°C) und unter Lichtausschluss gelagert werden.

Vorteilhaft kann die Hybridisierung und damit die Beschichtung mit den gewünschten Wirkstoffen kurz vor der Verwendung durch den Anwender erfolgen. Dies ermöglicht eine bestmögliche Flexibilität und kurzfristige Entscheidungen in der Wahl der Wirkstoffe sowie eine getrennte Lagerung von metallischem Gegenstand und Wirkstoffen.

Die Kopplung von Wirkstoffen an die Nukleinsäuren kann über an die Nukleinsäuren terminal gebundene funktionelle Gruppen, wie beispielsweise Aminoalkyl-, oder Mercaptoalkylgruppen, erfolgen (s. Agrawal, S. (Editor). 1994. Methods in Molecular Biology 26: Protocols for Oligonucleotide Conjugates, Humana Press Inc., Totowa, NJ, USA). Mit solchen funktionellen Gruppen, wie beispielsweise Aminohexyl-modifizierte Nukleinsäuren sind kommerziell erhältlich. Mit ebenfalls kommerziell erhältlichen Crosslinkern können Wirkstoffe über diese funktionellen Gruppen an die Nukleinsäuren gebunden werden. Kommerziell erhältlich sind auch mit Biotin terminal-modifizierte Nukleinsäuren. An diese können Avidin- oder Streptavidin-konjugierte Wirkstoffe binden.

In einer besonderen Ausführungsform des Nukleinsäure-beschichteten Gegenstands sind an die Nukleinsäurestränge organische oder anorganische Gruppen gebunden, die radioaktive Elemente enthalten. Ein so modifiziertes System ist für medizinisch-analytische Zwecke (Szintigrafien u.ä.) und für therapeutische Zwecke (lokale Strahlentherapie von Tumoren) geeignet.

Das erfindungsgemäße Verfahren grenzt sich durch folgende wesentliche Vorteile von bekannten Verfahren ab:
- Stabile Immobilisierung von Nukleinsäuren;
- gezielte Gestaltung einer gewünschten Einbautiefe der fixierten Nukleinsäure in die oberflächliche Oxidschicht ist möglich;
- es ist auch auf Nukleinsäurederivate anwendbar;
- nur ein Verfahrensschritt für die Immobilisierung der Nukleinsäuren;
- keine toxischen Chemikalien für Immobilisierung nötig;
- kostengünstig und einfach anwendbar, kontrollierbar, automatisierbar, für die industrielle Produktion geeignet;
- regiospezifischer terminaler Einbau der Nukleinsäuren;
- die Erzeugung reproduzierbarer und weitgehend identischer Immobilisierungszustände der Einzelmoleküle wird möglich. Dadurch wird ein sehr homogenes Verhalten der immobilisierten Nukleinsäuren bei nachfolgender Hybridisierung sowie eine Homogenität in der Stabilität der so erzeugten Hybride erreicht.

Bei einer Anwendung des erfindungsgemäß beschichteten metallischen Gegenstands für den Einsatz für Implantate in der Medizin, erlauben die genannten Variationsmöglichkeiten die alternative oder gleichzeitige Beschichtung des Implantats mit Substanzen unterschiedlicher biologischer Wirksamkeit, wie beispielsweise spezifischen Wachstumsfaktoren, Botenstoffen und Antikörpern. Vorteilhaft kann das Freisetzungsverhalten dieser Wirkstoffe durch Variationen in der Hybridstabilität beeinflusst werden. Die gleichzeitige oder zeitlich versetzte Freisetzung eines oder mehrerer Wirkstoffe von ein und derselben Oberfläche ist dadurch möglich.
Eine denkbare medizinische Anwendungsmöglichkeit ist die osteoinduktive Ausrüstung einer Implantatoberfläche für Knochenkontakt mit dem ,bone morphogenetic protein' Typ 4 (BMP-4) als an die Nukleinsäuren gekoppeltem Wirkstoff.
Eine andere Anwendungsmöglichkeit ist die Beschichtung der Implantatoberfläche mit dem die Zellproliferation und -differenzierung positiv beeinflussenden Wachstumsfaktor TGF-β (tissue growth factor beta) als an die Nukleinsäuren gekoppeltem Wirkstoff.

In einer weiteren Ausführungsform des Verfahrens sind an die komplementären Nukleinsäurestränge organische oder anorganische Gruppen gebunden, die radioaktive Elemente enthalten. Ein so modifiziertes System ist für medizinisch-analytische Zwecke (Szintigrafien u.ä.) und für therapeutische Zwecke (lokale Strahlentherapie von Tumoren) geeignet.

Anhand nachfolgender Ausführungsbeispiele wird die Erfindung näher erläutert. Eine Ausführungsvariante der erfindungsgemäßen Lösung wird in Ausführungsbeispiel 1 erläutert. Die weiteren Ausführungsbeispiele sind Negativ-Beispiele. Die in den folgenden Ausführungsbeispielen verwendeten DNA-Oligonukleotide wurden von der Firma SIGMA-ARK GmbH Darmstadt bezogen und haben folgende Sequenzen:

| | |
|---|---|
| T3E-5P: | ²⁻O₃PO-5'-CCA AAC CCG TCA ATC AAG TCT ACA CTG TTC-3' |
| T3E-5NH2: | H₂N -5'-CCA AAC CCG TCA ATC AAG TCT ACA CTG TTC-3' |
| S3E-Fl: | Fluorescein-HN-(CH₂)₆-5'-CAG TGT AGA CTT GAT-3' |

### Ausführungsbeispiel 1:

### 1a.) Vorbehandlung:

Eine zylindrische Probe aus TiAl6V4 mit einem Durchmesser von 10 mm und einer Dicke von 3 mm wird geschliffen, oxidpoliert und mit Ethanol gewaschen.

Dies so vorbereitete Probe aus TiAl6V4 wird als Substratelektrode in eine Drei-Elektroden-Anordnung mit einer Silber/Silberchloridelektrode als Bezugselektrode und einer Platin-Gegenelektrode in ein Well einer Zellkulturschale eingebracht.

Eine 400 nmol/Liter Lösung der 5'-phosphorylierten T3E-5P DNA in sterilem Acetatpuffer (0,2 mol/Liter, pH = 4) wird bei 95°C denaturiert und für mindestens 5 min auf Eis gestellt.

Nach dem Anschluss aller Elektroden an die Potenziostat-Galvanostat-Einheit werden 3 ml der vorbereiteten DNA-Lösung in das Well der Zellkulturschale gegeben und 15 min bei Raumtemperatur ohne eine Spannung anzulegen inkubiert.

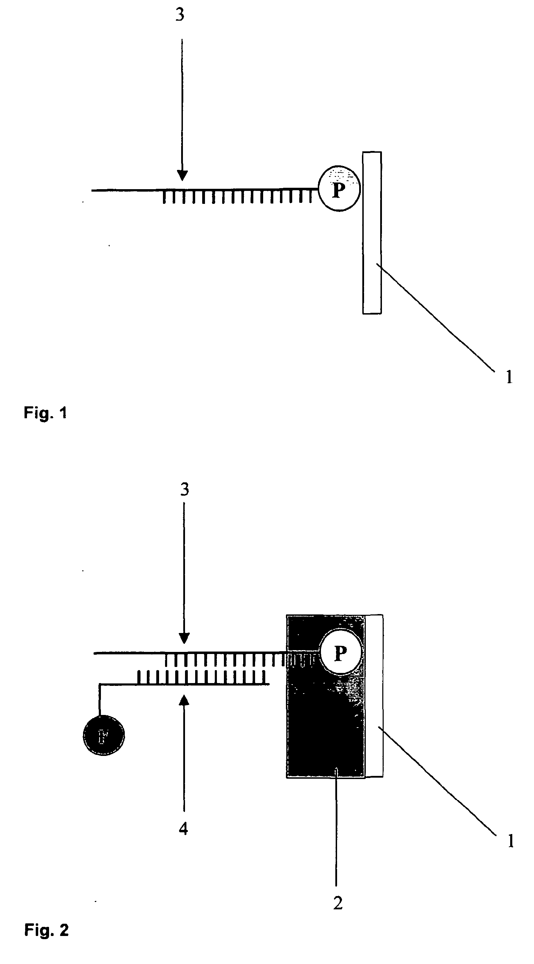

Wie schematisch in **Fig. 1** dargestellt werden durch diese Vorbehandlung einzelne Nukleinsäuremoleküle **3** an die aus einer dünnen Metall-Metalloxidschicht bestehende Substratoberfläche **1** metastabil fixiert. Der einfacheren Darstellung halber ist hier nur ein Nukleinsäurestrang dargestellt. Die Basen der Nukleinsäuren sind durch senkrechte Striche auf der durchgezogene Linie des Nukleinsäurerückgrates dargestellt.

### 1b.) Anodische Polarisation:

Anschließend wird die TiAl6V4-Probe mit einer Stromdichte von 76 µA*cm⁻² für 2 Minuten anodisch polarisiert. Das Potenzial wird auf maximal 10 V gegenüber der Ag/AgCl-Bezugselektrode begrenzt. Wird dieser Wert erreicht, so wird die Immobilisierung vorzeitig gestoppt.

Nach Abschluss der elektrochemischen Polarisation wird die Zellkulturschale mit der Titanprobe mit **3** ml sterilem Acetatpuffer (0,2 mol/Liter, pH = 4,0) und anschließend zweimal mit **3** ml sterilem Wasser gespült.

Durch die anodische Polarisation wird die bereits vorliegende Metalloxidschicht **1** um den Anteil **2** verdickt. Dieser durch die anodische Polarisation neuaufgebaute Anteil **2** der Metalloxidschicht umschließt dabei den terminalen Bereich der DNA-Moleküle (Fig 2).

### 1c.) Hybrisierung:

Zur Hybridisierung wird eine Lösung einer zum 3'-Terminus der durch die anodische Polarisation immobilisierten DNA-Stränge **3** (T3E-5P DNA) komplementären DNA (S3E-Fl DNA) **4** wie folgt vorbereitet:
Eine 40 nmol/Liter Lösung von S3E-Fl DNA in sterilem TRIS-HCl-Puffer (50 mmol/Liter; pH = 7,5) wird bei 95°C denaturiert und anschließend unverzüglich für mindestens 5 min auf Eis gestellt.

Mit 3 ml der so vorbereiteten Lösung wird die Probe 1 h inkubiert und anschlie-βend einmal mit TRIS-HCl-Puffer (50 mmol/Liter, pH = 7,5) und zweimal mit 3 ml sterilem Wasser gespült und steril und staubfrei luftgetrocknet.

Bei einer so behandelten Probe zeigt sich in der Fluoreszenzmikroskopie eine starke Fluoreszenz im grünen Wellenlängenbereich auf der Metalloberfläche. Dies belegt eine erfolgreiche, stabile Immobilisierung der 5'-phosphorylierten T3E-5P DNA an der Metall-Metalloxid-Oberfläche, sowie eine erfolgreiche Hybridisierung mit der komplementären S3E-Fl DNA.

**Fig. 2** zeigt schematisch die Oberfläche eines derart Nukleinsäure-beschichteten metallischen Gegenstands mit einer dünnen Metalloxidschicht **1,** in welche hier die 5'-terminalen Bereiche von Nukleinsäuremolekülen **3** mit den 5'-terminalen Phosphatgruppen **P** in eine durch anodische Polarisation aufgebaute 2. Metalloxidschicht **2** stabil eingebaut sind. Der einfacheren Darstellung halber ist hier nur ein Nukleinsäurestrang (in diesem Beispiel T3E-5P DNA) dargestellt. Durch komplementäre Basenpaarungen ist an dessen nicht in die Oxidschicht eingebauten Molekülbereich ein entsprechender Fluoreszenzmarkierter **F** Gegenstrang **4** (in diesem Beispiel S3E-Fl DNA) gebunden.

### Ausführungsbeispiel 2:

Eine metallische Probe aus TiAl6V4 wird, wie in Ausführungsbeispiel 2 beschrieben mit einer 5'-phosphorylierten T3E-5P DNA inkubiert (siehe 1a) und anschließend ohne dass eine anodische Polarisation (siehe 1 b) durchgeführt wird mit 3 ml sterilem Acetatpuffer (0,2 mol/Liter; pH = 4,0) und zweimal mit 3 ml sterilem Wasser gespült. Danach wird die Probe wie in Ausführungsbeispiel 1 beschrieben mit der, zum 3'-Terminus der T3E-5P DNA komplementären, fluoreszierenden S3E-Fl DNA inkubiert und gewaschen.

Fluoreszenzmikroskopisch konnte bei einer so behandelten Probe keine Fluoreszenz nachgewiesen werden. Da dieses Ausführungsbeispiel bis auf den Schritt der anodischen Polarisation exakt dem Ausführungsbeispiel 1 entspricht, zeigt es, dass im Gegensatz zu dem erfindungsgemäßen Verfahren (Ausführungsbeispiel 1) über eine reine Adsorption an die bereits vorhandene Metalloxidoberfläche keine stabile Bindung der DNA zu erreichen ist.

### Ausführungsbeispiel 3:

Die Durchführung entspricht der aus Ausführungsbeispiel 2 mit dem Unterschied, das die Probe aus TiAl6V4 nur mit der fluoreszierenden S3E-Fl DNA inkubiert wird und nicht zuvor mit der T3E-5P DNA.

Unter dem Fluoreszenzmikroskop kann bei einer so behandelten Probe keine Fluoreszenz nachgewiesen werden.
Ausführungsbeispiel 3 zeigt, dass auch Fluorescein-markierte Nukleinsäuren durch Adsorption nicht nachweisbar an einer auf der TiAl6V4-Oberfläche bereits vorhandenen Oxidschicht immobilisiert werden.

### Ausführungsbeispiel 4:

Die Durchführung entspricht Ausführungsbeispiel 1 mit dem Unterschied, dass zur anodischen Polarisation anstelle der 5'-phosphorylierten T3E-5P DNA eine Lösung der entsprechenden nicht phosphorylierten T3E-NH₂ verwendet wird.

Die so behandelte Probe wird anschließend ebenfalls wie in Ausführungsbeispiel 1 zur Hybridisierung mit einer S3E-Fl-DNA-Lösung inkubiert.

Unter dem Fluoreszenzmikroskop kann bei einer so behandelten Probe keine Fluoreszenz nachgewiesen werden. Dies zeigt, dass bei Nukleinsäuren, die nicht mit anonischen Gruppen, wie hier Phosphat, terminal modifiziert sind, kein Einbau in die durch die anodische Polarisation wachsende Oxidschicht stattfindet. Dieses Beispiel belegt, dass die in der T3E-NH₂-DNA vorhandenen Phosphatgruppen des Zucker-Phosphatrückgrates nicht für einen stabilen Einbau in die bei der elektrochemischen Polarisation neu gebildete Oxidschicht zur Verfügung stehen.

### Ausführungsbeispiel 5:

Die Durchführung entspricht Ausführungsbeispiel 1 mit dem Unterschied, dass zur anodischen Polarisation anstelle der 5'-phosphorylierten T3E-5P DNA eine Lösung der nicht phosphorylierten, fluoreszierenden S3E-Fl verwendet wird.

Eine erfolgreiche Immobilisierung sollte in diesem Fall direkt durch die Fluoreszenz des immobilisierten Einzelstranges nachweisbar sein. Auf den anschlie-βenden Hybridisierungsschritt mit Fluorescein-markierter DNA wird hier daher verzichtet.

Unter dem Fluoreszenzmikroskop kann bei einer so behandelten Probe keine Fluoreszenz nachgewiesen werden. Um zu ermitteln, ob die fehlende Fluoreszenz auf einen möglichen zu tiefen Einbau der Fluoresceinmoleküle in die Oberfläche zurückzuführen ist, wurde die Zusammensetzung der auf der Probenoberfläche gebildeten Oxidschicht mit Photoelektronenspektroskopie (XPS) analysiert. Bis in eine Tiefe von 5 nm konnte dabei kein Phosphor nachgewiesen werden. Das negative Ergebnis des Phosphor-Nachweises zeigt, dass keine Nukleinsäure gebunden wurde.
Die Ergebnisse der beschriebenen Ausführungsbeispiele und die Ergebnisse weiterer Vergleichsversuche werden in folgender Tabelle zusammengefasst:

**Tabelle 1:**

| Ausführungsbeispiel Nr. | Oligonukleotid¹ | pH¹ | anodische Polarisation² | Hybridisierung³ | Fluoreszenz | XPS⁴ |
|---|---|---|---|---|---|---|
| 1 | T3E-5P | 4 | +⁵ | + | + | n. d.⁵ |
| 2 | T3E-5P | 4 | -⁵ | + | - | n. d. |
| 3 | S3E-Fl | 4 | - | - | - | n. d. |
| 4 | T3E-NH₂ | 4 | + | + | - | n. d. |
| 5 | S3E-Fl | 4 | + | - | - | - |
| 6 | S3E-Fl | 7,5 | + | - | - | n. d. |
| 7 | S3E-Fl | 7,5 | - | - | - | n. d. |
| 8 | S3E-Fl | 7,5⁶ | - | - | - | n. d. |
| 9 | S3E-Fl | 7,5/4⁷ | + | - | - | - |

| | | | | | | |
|---|---|---|---|---|---|---|
| ¹ bei der Immobilisierung ² wie in Ausführungsbeispiel 1 beschrieben ³ mit S3E-Fl bei pH 7,5, wie in Ausführungsbeispiel 1 beschrieben ⁴ XPS = Photoelektronenspektroskopie, vergleiche Ausführungsbeispiel 5 ⁵ + = ja, - = nein, n. d. = nicht bestimmt ⁶ mit zusätzlich 10 mmol/Liter MgCl₂ in der Lösung ⁷ zuerst Inkubation mit zusätzlich 10 mmol/Liter MgCl₂ in der Lösung bei pH 7.5 und anschließende anodische Polarisation bei pH 4 | | | | | | |

Die Vergleichsversuche 6 bis 9 wurden entsprechend den detailliert beschriebenen Ausführungsbeispielen 1 bis 5 jeweils mit einer Probe aus TiAl6V4 durchgeführt, mit den in der Tabelle angegebenen Abweichungen. Die Bedingungen unter denen die Immobilisierung, d. h. die Inkubation mit der DNA aus Spalte 2 und der sich gegebenenfalls anschließenden anodischen Polarisation sind in der Spalte 3 angeben. Spalten 4 und 5 geben an, ob eine anodische Polarisation und eine Hybridisierung mit einer komplementären DNA erfolgte. In den Spalten 6 und 7 sind die Ergebnisse der Fluoreszenzmikroskopie bzw. der Photonenelektronenspektroskopie (XPS) aufgeführt.
Zusammengefasst geht aus den Ausführungsbeispielen und der Parameterzusammenstellung in Tabelle 1 hervor, dass
- Nukleinsäuren durch eine Adsorption nicht nachweisbar auf einer Ti-Al6V4-Oberfläche immobilisiert werden (Ausführungsbeispiele 2, 3, 7 und 8),
- 5'-phosphorylierte Nukleinsäuren durch anodische Polarisation bei pH 4,0 stabil auf einer TiAl6V4-Oberfläche immobilisiert werden und die 3'-terminalen Molekülbereiche frei zugänglich für anschließende Hybridisierungen sind (Ausführungsbeispiel 1).
- Nukleinsäuren, die nicht terminal phosphoryliert sind, durch anodische Polarisation bei pH 4,0 nicht nachweisbar auf einer TiAl6V4-Oberfläche immobilisiert werden (Ausführungsbeispiele 4, 5, 6 und 9).

### Aufstellung der verwendeten Bezugszeichen:

- 1: dünne Oxidschicht an der Substratoberfläche
- 2: durch anodische Polarisation neu gebildete Oxidschicht
- 3: zu immobilisierender Nukleinsäurestrang
- 4: komplementärer Nukleinsäurestrang
- F: Fluorescein
- P: Phosphat

## Patentansprüche

1. Metallischer Gegenstand dessen Oberfläche mit einer dünnen Metalloxidschicht überzogen und mit Nukleinsäuren beschichtet ist, **dadurch gekennzeichnet, dass** 5'- oder 3'-terminale Molekülbereiche der Nukleinsäuren durch Integration in eine wachsende Metalloxidschicht stabil in die Metalloxidschicht eingebaut sind, wobei die eingebauten 5' oder 3' terminalen Bereiche der Nukleinsäuren anionischen Gruppen tragen und wobei durch den regiospezifischen Einbau der terminalen Molekülbereiche eine frei bewegliche Orientierung der nicht-eingebauten Bereiche der Nukleinsäuren zur Metalloberfläche erreicht wird.

2. Gegenstand nach Anspruch 1, **dadurch gekennzeichnet, dass** die anionischen Gruppen Phosphat- oder Phosphonat- oder Sulfonatgruppen sind.

3. Gegenstand nach Anspruch 1 oder 2, **dadurch gekennzeichnet, dass** das Metall aus einem Ventilmetall oder einer Ventilmetalllegierung besteht.

4. Gegenstand nach einem der Ansprüche 1 bis 3, **dadurch gekennzeichnet, dass** das Metall aus Aluminium oder Titan oder Tantal oder Zirkonium oder Niob oder einer Legierung, einschließlich intermetallischer Phasen, eines oder mehrerer dieser Metalle besteht.

5. Gegenstand nach einem der Ansprüche 1 bis 4, **dadurch gekennzeichnet, dass** die Nukleinsäuren Desoxyribonukleinsäuren (DNA) oder Ribonukleinsäuren (RNA) oder Peptidische Nukleinsäuren (PNA) oder Locked Nucleic Acids (LNA) oder gemischte Moleküle aus diesen sind.

6. Gegenstand nach Anspruch 5, **dadurch gekennzeichnet, dass** die Nukleinsäuren zusätzliche Modifikationen des Zucker-Phosphat-Rückgrates, wie Phosphothioat- oder O-Methyl-gruppen und/oder unkonventionelle Basen, wie Inosin, enthalten.

7. Gegenstand nach einem der Ansprüche 1 bis 6, **dadurch gekennzeichnet, dass** die Nukleinsäuren zumindest partiell als Einzelstränge vorliegen.

8. Gegenstand nach Anspruch 7, **dadurch gekennzeichnet, dass** an den Einzelsträngen weitere Nukleinsäurestränge durch komplementäre Basenpaarung gebunden sind.

9. Gegenstand nach Anspruch 8, **dadurch gekennzeichnet, dass** der an der Metall-Metalloxid-Oberfläche immobilisierte und der komplementäre Strang kovalent verbunden sind.

10. Gegenstand nach Anspruch 8 oder 9, **dadurch gekennzeichnet, dass** an die komplementären Nukleinsäurestränge Wirkstoffe, wie anorganische oder organische oder biochemische Moleküle oder Zell- oder Gewebekomponenten gebunden sind.

11. Gegenstand nach Anspruch 10, **dadurch gekennzeichnet, dass** an die komplementären Nukleinsäurestränge organische oder anorganische Gruppen gebunden sind, die radioaktive Elemente enthalten.

12. Verfahren zur Herstellung eines metallischen Gegenstands mit einer Beschichtung, bestehend aus einer dünnen Metalloxidschicht und Nukleinsäuren, wobei die 5'- oder 3'-terminalen Molekülbereiche der Nukleinsäuren anionische Gruppen tragen und elektrochemisch um die 5'- oder 3'-terminale Molekülbereiche herum eine Metalloxidschicht aufgebaut wird, wobei eine adsorptive Anlagerung und ein anschließender Einbau der terminalen Molekülbereiche durch anodische Polarisation bei pH 3 bis 6 erfolgt.

13. Verfahren nach Anspruch 12, **dadurch gekennzeichnet, dass** für das erreichte Potenzial ein Wert zwischen 2 und 200 V_{SCE} gewählt wird.

14. Verfahren nach Anspruch 12 oder 13, **dadurch gekennzeichnet, dass** an ein mit einzelsträngigen Nukleinsäuren beschichtetes Metall anschließend eine Hybridisierung von komplementären Nukleinsäuresträngen erfolgt, wobei der pH-Wert der Hybridisierungslösung zwischen pH 4 bis 10 und die Ionenstärke im Bereich von 0,1 bis 1,5 mol/Liter gewählt wird.

15. Verfahren nach Anspruch 14 **dadurch gekennzeichnet, dass** die Hybridisierung bei einem pH-Wert im Bereich von pH 5,5 bis 8,5 durchgeführt wird.

16. Verfahren nach Anspruch 14 oder 15, **dadurch gekennzeichnet, dass** an die komplementären Nukleinsäurestränge Wirkstoffe, wie anorganische oder organische oder biochemische Moleküle oder Zell- oder Gewebekomponenten gebunden sind.

17. Verfahren nach Anspruch 16, **dadurch gekennzeichnet, dass** an die komplementären Nukleinsäurestränge organische oder anorganische Gruppen gebunden sind, die radioaktive Elemente enthalten.

18. Verwendung eines Gegenstandes nach einem der Ansprüche 1 bis 11 als Material für die Herstellung von Implantaten für die Medizin.

## Claims

1. Metallic object the surface of which is covered with a thin metal oxide layer and coated with nucleic acids, **characterized in that** the molecular areas at the 5'-terminus or 3'-terminus of the nucleic acids are incorporated stably into the metal oxide layer by integration into a growing metal oxide layer, wherein the incorporated 5'-terminal or 3'-terminal areas of the nucleic acids comprise anionic groups and wherein by regiospecifical incorporation of the terminal molecular areas a freely movable orientation of the non-incorporated areas of the nucleic acids relative to the metal surface is achieved.

2. Object according to claim 1, **characterized in that** the anionic groups are phosphate or phosphonate or sulfonate groups.

3. Object according to claim 1 or 2, **characterized in that** the metal consists of a valve metal or a valve metal alloy.

4. Object according to one of claims 1 to 3, **characterized in that** the metal consists of aluminum or titanium or tantalum or zirconium or niobium, or an alloy, including intermetallic phases, of one or more of these metals.

5. Object according to one of claims 1 to 4, **characterized in that** the nucleic acids are desoxyribonucleic acids (DNA) or ribonucleic acids (RNA) or peptide nucleic acids (PNA) or locked nucleic acids (LNA) or mixed molecules thereof.

6. Object according to claim 5, **characterized in that** the nucleic acids contain additional modifications of the sugar phosphate backbone, like phosphothioate- or O-methyl groups and/or unconventional bases, like inosine.

7. Object according to one of claims 1 to 6, **characterized in that** the nucleic acids are present at least partially as single strands.

8. Object according to claim 7, **characterized in that** additional nucleic acid strands are bonded to the single strands by complementary base pairing.

9. Object according to claim 8, **characterized in that** the strand immobilized on the metal-metaloxide-surface and the complementary strain are covalently bound to each other.

10. Object according to claim 8 or 9, **characterized in that** active ingredients such as inorganic or organic or biochemical molecules or cell components or tissue components are bound to the complementary nucleic acid strands.

11. Object according to claim 10, **characterized in that** organic or inorganic groups that contain radioactive elements are bound to the complementary nucleic acid strands.

12. Method for manufacturing a metallic object with a coating that consists of a thin metal oxide layer and nucleic acids, wherein the molecular areas at the 5'-terminus or 3'-terminus of the nucleic acids comprise anionic groups and a metal oxide layer is built up around the 5'- and 3'-terminal molecular areas by means of electrochemistry, wherein an adsorptive attachment and a subsequent incorporation of the terminal molecular areas is realized by anodic polarization at pH 3 to 6.

13. Method according to claim 12, **characterized in that** a value between 2 and 200 V_{SCE} is selected for the achieved potential.

14. Method according to claim 12 or 13, **characterized in that** a subsequent hybridization with complementary nucleic acid strands is carried out on the metal coated with single stranded nucleic acid molecules, whereby the pH value of the hybridization solution is selected between pH 4 and 10, and the ionic strength is in a range of 0.1 to 1.5 mol/liter.

15. Method according to claim 14, **characterized in that** the hybridization is performed at a pH value in a range of pH 5.5 to 8.5.

16. Method according to claim 14 or 15, **characterized in that** active ingredients such as inorganic or organic or biochemical molecules or cell components or tissue components are bound to the complementary nucleic acid strands.

17. Method according to claim 16, **characterized in that** organic or inorganic groups that contain radioactive elements are bound to the complementary nucleic acid strands.

18. Use of an object according to one of claims 1 to 11 as material for the manufacturing of implants for medicine.

## Revendications

1. Objet métallique dont la surface est revêtue d'une mince couche d'oxyde métallique et enduite d'acides nucléiques, **caractérisé en ce que** les zones moléculaires 5'-terminales ou 3'-terminales des acides nucléiques sont incorporées de façon stable dans la couche d'oxyde métallique par intégration dans une couche d'oxyde métallique croissante, où les zones 5'- ou 3'-terminales incorporées des acides nucléiques portent des groupes anioniques et où, par l'incorporation régiospécifique des zones terminales de molécules, on atteint une orientation librement mobile des zones non incorporées des acides nucléiques par rapport à la surface métallique.

2. Objet selon la revendication 1, **caractérisé en ce que** les groupes anioniques sont des groupes phosphate ou phosphonate ou sulfonate.

3. Objet selon la revendication 1 ou 2, **caractérisé en ce que** le métal se compose d'un métal de soupape ou d'alliage de métal de soupape.

4. Objet selon l'une des revendications 1 à 3, **caractérisé en ce que** le métal se compose d'aluminium ou de titane ou de tantale ou de zirconium ou de niobium ou d'un alliage, y compris des phases intermétalliques, d'un ou de plusieurs de ces métaux.

5. Objet selon l'une des revendications 1 à 4, **caractérisé en ce que** les acides nucléiques sont des acides désoxyribonucléiques (ADN) ou des acides ribonucléiques (ARN) ou des acides nucléiques peptidiques (ANP) ou des acides nucléiques bloqués (LNA) ou des molécules mixtes de ceux-ci.

6. Objet selon la revendication 5, **caractérisé en ce que** les acides nucléiques contiennent des modifications supplémentaires du squelette sucre-phosphate, comme des groupes phosphothioate ou O-méthyle et/ou des bases non conventionnelles, telles que l'inosine.

7. Objet selon l'une des revendications 1 à 6, **caractérisé en ce que** les acides nucléiques se présentent au moins partiellement en tant que simples brins.

8. Objet selon la revendication 7, **caractérisé en ce que** d'autres brins d'acide nucléique sont liés aux simples brins par appariement de base complémentaire.

9. Objet selon la revendication 8, **caractérisé en ce que** le brin immobilisé au niveau de la surface métal-oxyde métallique et le brin complémentaire sont liés de façon covalente.

10. Objet selon la revendication 8 ou 9, **caractérisé en ce que** des substances actives comme des molécules inorganiques ou organiques ou biochimiques ou des composants cellulaires ou de tissu sont liés aux brins complémentaires d'acide nucléique.

11. Objet selon la revendication 10, **caractérisé en ce que** des groupes organiques ou inorganiques qui contiennent des éléments radioactifs sont liés aux brins complémentaires d'acide nucléique.

12. Procédé de fabrication d'un objet métallique comportant un revêtement composé d'une couche mince d'oxyde métallique et d'acides nucléiques, où les zones de molécules 5'- et 3'-terminales des acides nucléiques portent des groupes anioniques et une couche d'oxyde métallique est formée par voie électrochimique autour des zones de molécules 5' ou 3'-terminales, où un dépôt par adsorption et une incorporation consécutive des zones terminales des molécules se faisant par polarisation anodique à un pH compris entre 3 et 6.

13. Procédé selon la revendication 12, **caractérisé en ce que** pour le potentiel atteint, ou sélectionne une valeur comprise entre 2 et 200 V_{SCE}.

14. Procédé selon la revendication 12 ou 13, **caractérisé en ce qu'**au niveau d'un métal enduit d'acides nucléiques simples brins, on réalise ensuite une hybridation de brins complémentaire d'acide nucléique, la valeur de pH de la solution d'hybridation étant sélectionnée entre pH 4 et pH 10 et la force ionique étant sélectionnée dans la plage de 0,1 à 1,5 mol/litre.

15. Procédé selon la revendication 14, **caractérisé en ce que** l'hybridation est conduite à une valeur de pH comprise dans la plage de pH 5,5 à pH 8,5.

16. Procédé selon la revendication 14 ou 15, **caractérisé en ce que** des substances actives comme des molécules inorganiques ou organiques ou biochimiques ou des composants cellulaires ou de tissu sont liés aux brins complémentaires d'acide nucléique.

17. Procédé selon la revendication 16, **caractérisé en ce que** des groupes organiques ou inorganiques qui contiennent des éléments radioactifs sont liés aux brins complémentaires d'acide nucléique.

18. Utilisation d'un objet selon l'une des revendications 1 à 11 en tant que matériau destiné à la fabrication d'implants pour la médecine.
